# EUROPEAN PATENT SPECIFICATION

(11) **EP 4 176 918 B1**
(45) Date of publication and mention of the grant of the patent: **27.08.2025**
(21) Application number: 20944468.6
(22) Date of filing: 06.07.2020
(51) Int. Cl.: A61M 25/00, A61B 17/3207, A61B 17/22

(54) **CATHETER**
KATHETER
CATHÉTER

(43) Date of publication of application: 10.05.2023
(73) Proprietor: ASAHI INTECC CO., LTD., Seto-shi, Aichi, 489-0071 (JP)
(72) Inventor: HARNMONTRI, Nawaporn, Seto-shi, Aichi 489-0071 (JP)
(74) Representative: Winter, Brandl - Partnerschaft mbB
(86) International application number: PCT/JP2020/026490
(87) International publication number: WO 2022/009287

(56) References cited:
- WO-A1-2018/181962
- JP-A- 2004 501 672
- JP-A- 2005 296 078
- JP-A- 2008 539 946
- JP-A- 2014 195 556
- JP-A- 2015 208 425
- JP-A- 2018 050 722
- JP-A- 2018 158 008
- US-A- 5 527 298
- US-A1- 2002 156 496
- US-A1- 2007 118 204
- US-A1- 2007 239 109
- US-A1- 2012 209 176
- US-A1- 2012 271 277
- US-A1- 2015 306 347
- US-B2- 7 025 751

## Description

### TECHNICAL FIELD

The present invention relates to a catheter.

### BACKGROUND ART

For example, catheters for improving the blood flow by removing occlusions that block blood vessels, such as a chronic total occlusion (CTO), are known.

Among such catheters, there is known a catheter provided with a metal distal tip at a distal end for passing the catheter through a hard lesion site (see, for example, Japanese Unexamined Patent Application Publication No. 2018-519957).

US 5 527 298 A discloses a guidewire capable of penetrating a vascular occlusion, and provides a guidewire assembly wherein a stylet can be removably inserted into the length of the guidewire lumen to thereby enhance the guidewire structure.

In US 2002/156496 A1, a catheter is configured to carry one or more stents and to have an inflatable balloon for expanding a stent surrounding the balloon.

US 7 025 751 B2 provides a catheter for distal removal of thrombi by suction, specifically designed for percutaneous coronary surgery operations, and which is easy and quick to manipulate and to position.

US 2012/209176 A1 describes a catheter capable of passing through a vascular occlusion.

US 2007/239109 A1 discloses a catheter for the dilation of stenotic lumenal occlusions, as well as methods of use for the device.

Further, US 2015/306347 A1 describes a catheter, which is capable of passing by or through a hard lesion, in accordance with the preamble of claim 1.

### SUMMARY OF INVENTION

### TECHNICAL PROBLEM

It is required for catheters intended to be passed through a hard lesion that the catheter is easily passed through a lesion site and the damage to the catheter can be prevented when the catheter is passed through the lesion site.

An object of the present invention is to provide a catheter suitable for being easily passed through a hard lesion site and for preventing damage to blood vessels.

### SOLUTION TO OBJECT

This object is solved by the subject-matter of the independent claim. Further aspects are disclosed in the subclaims.

### ADVANTAGEOUS EFFECT OF INVENTION

According to the present invention, it is possible to provide a catheter suitable to be passed through a hard site.

### BRIEF DESCRIPTION OF DRAWINGS

FIG. 1 is a diagram of a configuration of a catheter according to a first embodiment (not covered by the subject-matter of the claims).
FIG. 2 is a diagram of a configuration of the vicinity of a distal end of the catheter illustrated in FIG. 1.
FIG. 3 is a diagram of a configuration of the vicinity of the distal end of a catheter according to a second embodiment (not covered by the subject-matter of the claims).
FIG. 4 is a diagram of a configuration of the vicinity of the distal end of a catheter according to a third embodiment, which is an embodiment of the present invention.
FIG. 5 is a diagram of a configuration of a distal tip of a catheter according to a fourth embodiment (not covered by the subject-matter of the claims).
FIG. 6 is a diagram of a configuration of a distal tip of a catheter according to a fifth embodiment (not covered by the subject-matter of the claims).

### DESCRIPTION OF EMBODIMENTS

Catheters according to several embodiments will be described with reference to the drawings (not all embodiments are covered by the subject-matter of the claims).

As used herein, the terms "distal end side" and "distal end direction" refer to a side and a direction where a distal tip is positioned with respect to a hollow shaft in a direction along a longitudinal direction of a catheter (direction along an axial direction of the hollow shaft). The terms "proximal end side" and "proximal end direction" refer to a side and a direction that are opposite to the distal end side and the distal end direction at a side and in a direction along a direction along the longitudinal direction of the catheter. Further, the term "distal end" refers to an end portion at the distal end side in any member or site, and the term "proximal end" refers to an end portion at the proximal end side in any member or site, respectively.

### First Embodiment (not covered by the subject-matter of the claims)

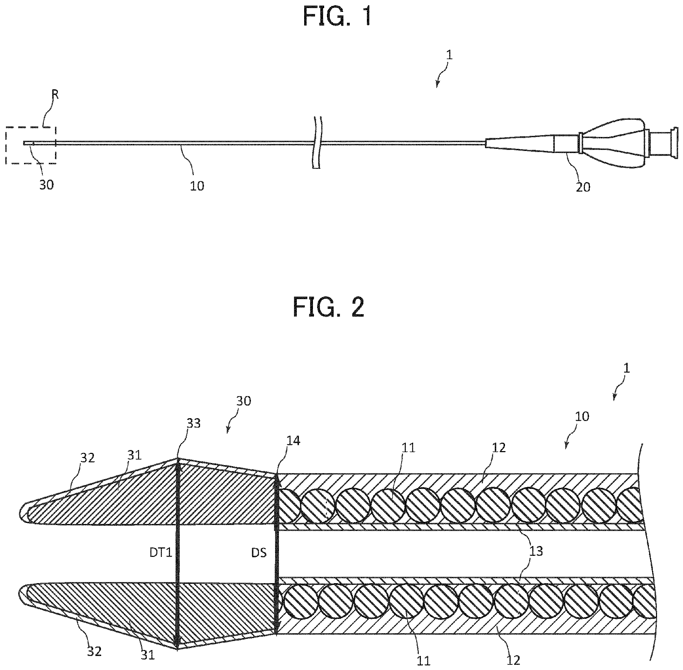

FIG. 1 is a diagram of a configuration of a catheter according to a first embodiment (not covered by the subject-matter of the claims). A catheter 1 includes a shaft 10, a connector 20 provided on the proximal end side of the shaft 10, and a distal tip 30 connected to the distal end side of the shaft 10.

FIG. 2 illustrates a configuration of the vicinity of a distal end of the catheter illustrated in FIG. 1, and more specifically, is an enlarged view of a cross section of a region R. The shaft 10 is a hollow member and includes a coil body 11, an outer layer 12, and an inner layer 13.

The coil body 11 is an example of a reinforcing body that reinforces the shaft 10, and is formed by spirally winding a metal wire. The coil body 11 may be formed by spirally winding one wire (solid wire coil), or may be formed by spirally winding a plurality of wires (stranded coil). Considering a rotation force of the catheter 1, the coil body 11 is preferably a stranded coil. The metal material of the wire of the coil body 11 may be stainless steel (SUS304, SUS316, and the like), gold, platinum, tungsten, platinum, nickel, alloys containing these elements, and the like.

The outer layer 12 is a layer formed of a resin, for example, and is provided to cover an outer periphery of the coil body 11. The resin material forming the outer layer 12 is not particularly limited, and may be, for example, a polyamide, a polyamide elastomer, a polyester, polyurethane, and the like.

The inner layer 13 is a layer formed of a resin, for example, and is provided to cover an inner periphery of the coil body 11. The resin material forming the inner layer 13 is not particularly limited, but in consideration of slidability between the shaft 10 and an instrument (such as a guide wire) to be inserted inside the shaft 10, PTFE (polytetrafluoroethylene) is preferable.

The distal tip 30 that is hollow and made of a metal, is connected to a distal end portion 14 of the shaft 10. The distal tip 30 includes a main body portion 31 and a coating layer 32.

The main body portion 31 is formed of a metal material. The metal material forming the main body portion 31 is not particularly limited, and may be stainless steel (SUS304, SUS316, and the like), gold, platinum, tungsten, platinum, nickel, alloys containing these elements, and the like. The distal tip 30 may be formed of a radiopaque metal material so that it is possible to grasp a position of a distal end of the catheter 1 in a radioscopic image.

The coating layer 32 is a layer formed of a resin, for example, and is provided to cover an outer periphery of the main body portion 31. The resin material forming the coating layer 32 is not particularly limited, and may be, for example, a polyamide, a polyamide elastomer, a polyester, polyurethane, and the like.

The distal tip 30 includes, between the distal end of the distal tip 30 and the distal end portion 14 of the shaft 10, an enlarged diameter portion 33 where an outer diameter in a direction perpendicular to the axial direction (longitudinal direction) of the shaft 10 is largest. An outer diameter DT1 of the enlarged diameter portion 33 is larger than an outer diameter DS of the distal end portion 14 of the shaft 10. An outer diameter of the main body portion 31 in the enlarged diameter portion 33 is also larger than the outer diameter DS of the distal end portion 14 of the shaft 10.

The distal tip 30 is formed to have a tapered shape in which the outer diameter decreases from the enlarged diameter portion 33 toward the distal end, and the outer diameter decreases from the enlarged diameter portion 33 toward the distal end portion 14 of the shaft 10. A rear end of the distal tip 30 has the same outer diameter as an outer diameter D2, and an outer peripheral edge of the rear end of the distal tip 30 is connected to an outer peripheral edge of the distal end portion 14 of the shaft 10. The shaft 10 and the distal tip 30 are connected to have a continuous outer periphery.

The distal tip 30 and the shaft 10 are formed so that a rear end surface of the distal tip 30 and a distal end surface of the shaft 10 contact, at least in part, each other, and the rear end surface of the distal tip 30 and the distal end surface of the shaft 10 are joined to each other. As illustrated in FIG. 2, the coil 11 and the main body portion 31 may be joined by welding at a surface where the metal materials contact each other. Moreover, the coating layer 32 and the outer layer 12 may be joined by thermal welding at a site where the resins contact each other.

If the catheter 1 is advanced through a hard lesion (for example, a calcified lesion), the shaft 10 passes through a portion through which the enlarged diameter portion 33 of the distal tip 30 passes. In the catheter 1, the outer diameter of the distal end portion 14 of the shaft 10 is smaller than the outer diameter of the enlarged diameter portion 33, so that a contact area between the lesion site and the shaft 10 is reduced. By reducing the contact area, it is possible to appropriately reduce the probability that the shaft 10 gets caught in the lesion or is damaged by the lesion. In addition, by reducing the contact area, it is possible to reduce the contact resistance by the shaft 10 when advancing the catheter 1, and to improve the pushing force (pushability) of the catheter 1.

### Second Embodiment (not covered by the subject-matter of the claims)

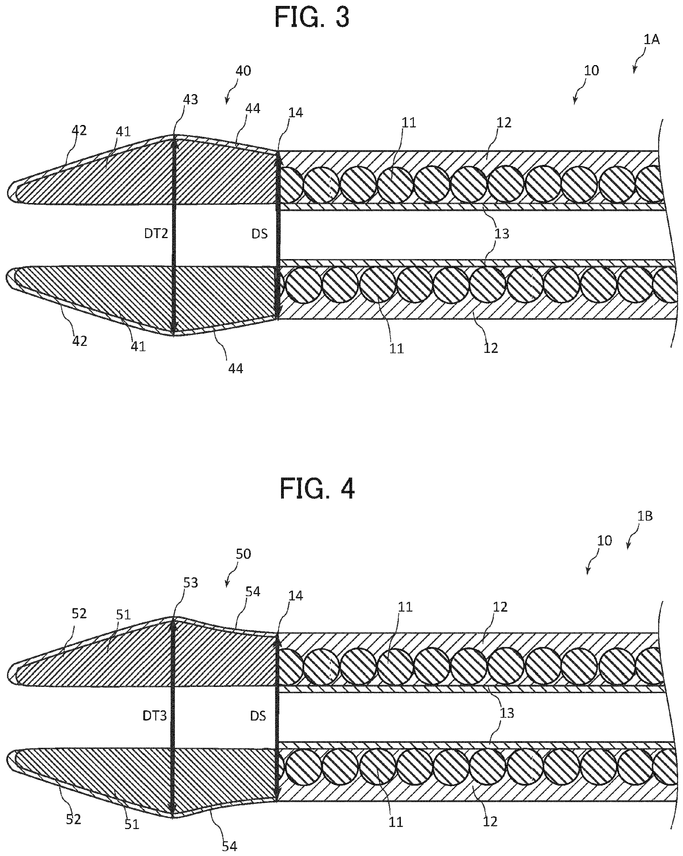

FIG. 3 is a diagram of a configuration of the vicinity of a distal end of a catheter according to a second embodiment (not covered by the subject-matter of the claims), and illustrates a cross-section of the distal end of the catheter illustrated in FIG. 2. A catheter 1A according to the second embodiment differs from the catheter 1 in the shape of the distal tip. Portions similar to those of the above-described embodiment are given the same reference symbols, and duplicated description is omitted. A distal tip 40 according to the second embodiment includes a main body portion 41 and a coating layer 42. The main body portion 41 and the coating layer 42 are similar to the main body portion 31 and the coating layer 32, except that the shapes are different.

The distal tip 40 includes, between the distal end and the distal end portion 14 of the shaft 10, an enlarged diameter portion 43 where the outer diameter is largest. An outer diameter DT2 at the enlarged diameter portion 43 is larger than the outer diameter DS of the distal end portion 14 of the shaft 10. The outer diameter of the main body portion 41 at the enlarged diameter portion 43 is also larger than the outer diameter DS of the distal end portion 14 of the shaft 10. The distal tip 40 includes an outer peripheral surface 44 extending from the distal end side of the enlarged diameter portion 43 to the distal end portion 14 of the shaft 10 via the enlarged diameter portion 43. As illustrated in FIG. 3, the outer peripheral surface 44 is formed with an outwardly convex curved surface having an inclination gradually changing in a longitudinal sectional view of the catheter 1A, that is, a gently curved surface. The inclination refers to an amount of change of the outer peripheral surface 44 in a radial direction of the catheter 1A, with respect to the length in the axial direction of the catheter 1A, in a longitudinal sectional view.

According to the catheter 1A according to the present embodiment, in addition to the effects of the first embodiment (not covered by the subject-matter of the claims), when the catheter 1A is advanced toward the distal end side, it is possible to appropriately prevent damage to normal blood vessels that contact the enlarged diameter portion 43 of the distal tip 40 and the outer peripheral surface of the catheter 1A on the proximal end side of the enlarged diameter portion 43.

### Third Embodiment

FIG. 4 is a diagram of a configuration of the vicinity of a distal end of a catheter according to a third embodiment, which is an embodiment of the present invention, and illustrates a cross-section of the distal end of the catheter illustrated in FIG. 2. A catheter 1B according to the third embodiment differs from the catheter 1 in the shape of the distal tip. Portions similar to those of the above-described embodiment are given the same reference symbols, and duplicated description is omitted. A distal tip 50 according to the third embodiment includes a main body portion 51 and a coating layer 52. The main body portion 51 and the coating layer 52 are similar to the main body portion 31 and the coating layer 32, except that the shapes are different.

The distal tip 50 includes, between the distal end and the distal end portion 14 of the shaft 10, an enlarged diameter portion 53 where the outer diameter is largest. An outer diameter DT3 at the enlarged diameter portion 53 is larger than the outer diameter DS of the distal end portion 14 of the shaft 10. The outer diameter of the main body portion 51 at the enlarged diameter portion 53 is also larger than the outer diameter DS of the distal end portion 14 of the shaft 10. In the distal tip 50, an outer peripheral surface 54 is formed with a more outwardly concave curved surface at the distal end portion 14 of the shaft 10 than at the enlarged diameter portion 53.

If the catheter 1B is advanced through a hard lesion (for example, a calcified lesion), in a portion through which the enlarged diameter portion 53 of the distal tip 50 passes, it is possible to more effectively reduce the contact of the outer peripheral surface 54 at the proximal end side of the enlarged diameter portion 53 with the lesion. Thus, it is possible to appropriately reduce the probability that the outer peripheral surface 54 gets caught in the lesion. It is possible to reduce the probability that the enlarged diameter portion 53 of the distal tip 50 contacts the lesion, and thus, it is possible to reduce the contact resistance when advancing the catheter 1B, and to improve the pushing force (pushability) of the catheter 1B. If the catheter 1B is advanced toward the distal end side, the probability that the outer peripheral surface 54 of the distal tip 50 contacts a normal blood vessel can be reduced, and it is possible to appropriately prevent damage to blood vessels.

### Fourth Embodiment (not covered by the subject-matter of the claims)

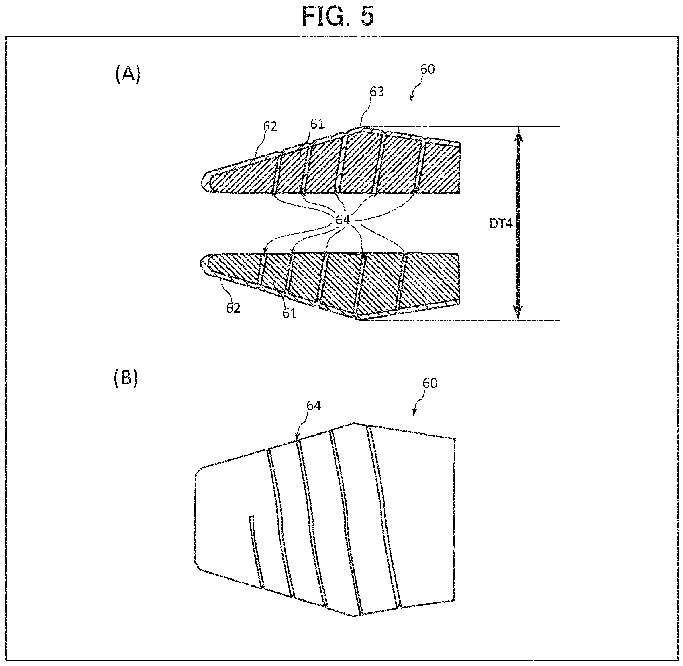

FIG. 5 is a diagram of a configuration of a distal tip of a catheter according to a fourth embodiment (not covered by the subject-matter of the claims), in which (A) is a cross-sectional view of the distal tip and (B) is a side view of the distal tip. In contrast to the distal tip 30 according to the first embodiment (not covered by the subject-matter of the claims), a distal tip 60 according to the fourth embodiment is formed with a slit 64. The distal tip 60 includes a main body portion 61 and a coating layer 62. The main body portion 61 is similar to the main body portion 31, except that the main body portion 61 is formed with the slit 64. The coating layer 62 is similar to the coating layer 32, except that the coating layer 62 penetrates into the slit 64.

In the main body portion 61, as illustrated in FIG. 5(B), the slit 64 is formed in a spiral shape. The slit 64 is provided to improve the flexibility of the main body portion 61 by cutting into the main body portion 61. In the present embodiment, the slit 64 is formed in a spiral shape, and thus, the distal tip 60 can be bent equally easily in any direction. Since a pitch of the slit 64 is formed to decrease toward the distal end, a distal end region of the distal tip 60 is particularly flexible. Therefore, it is possible to reduce the risk of perforating a blood vessel with the distal tip 60.

As illustrated in FIG. 5(A), the distal tip 60 is configured such that the resin of the coating layer 62 penetrates inside the slit 64 of the main body portion 61.

The distal tip 60 is provided with the slit 64, so that the distal tip 60 can be easily bent while maintaining the hardness of the distal tip 60. The outer peripheral surface of the distal tip 60 is covered with the coating layer 62, and thus, it is possible to smooth the surface of the distal tip 60. As a result, the catheter provided with the distal tip 60 can be easily passed through a hard lesion and exhibits good followability even in a tortuous blood vessel such as a peripheral blood vessel.

In the distal tip 60, the resin of the coating layer 62 penetrates inside the slit 64, so that it is possible to firmly fix the coating layer 62 to the main body portion 61. Therefore, it is possible to appropriately prevent the coating layer 62 from detaching from the main body portion 61 due to friction with the outside. Thus, the smoothness of the outer peripheral surface of the distal tip 60 can be maintained, even if the distal tip 60 is passed through a hard lesion.

### Fifth Embodiment (not covered by the subject-matter of the claims)

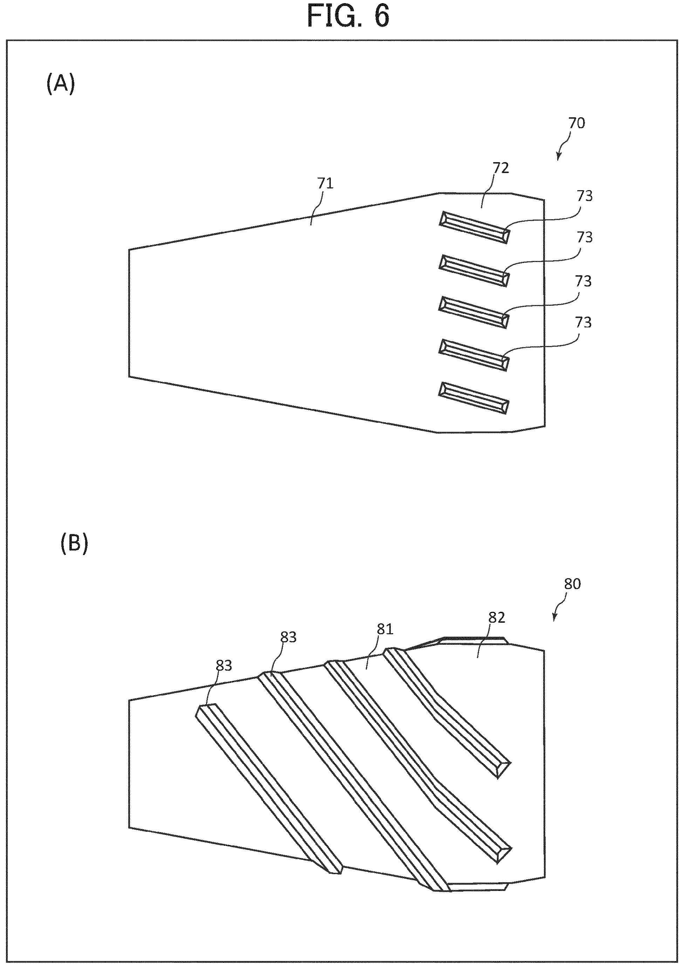

FIG. 6 is a diagram of a configuration of a distal tip of a catheter according to a fifth embodiment (not covered by the subject-matter of the claims), in which (A) is a side view of the distal tip and (B) is a side view of a distal tip according to a modification modified from the configuration illustrated in (A).

A distal tip 70 includes a main body portion 71. The main body portion 71 is similar to the main body portion 31, except that the shapes are different. The distal tip 70 includes, between the distal end and the distal end portion 14 of the shaft 10, an enlarged diameter portion 72 where the outer diameter is largest. The enlarged diameter portion 72 has a predetermined width in the axial direction of the shaft 10. The outer diameter at the enlarged diameter portion 72 is larger than the outer diameter DS of the distal end portion 14 of the shaft 10. A protruding portion 73 extending spirally and protruding radially outward of the main body portion 71 is provided on a surface (outer peripheral surface) of the main body portion 71 of the distal tip 70. The protruding portion 73 is provided only within the range of the enlarged diameter portion 72. According to the distal tip 70, since the distal tip 70 is provided with the protruding portion 73, it is possible to improve the penetrability into a hard lesion site.

The distal tip may be formed as a distal tip 80 illustrated in FIG. 6(B). The distal tip 80 includes a main body portion 81. The main body portion 81 is similar to the main body portion 31, except that the shapes are different. The distal tip 80 includes, between the distal end and the distal end portion 14 of the shaft 10, an enlarged diameter portion 82 where the outer diameter is largest. The enlarged diameter portion 82 has a predetermined width in the axial direction of the shaft 10. The outer diameter at the enlarged diameter portion 82 is larger than the outer diameter DS of the distal end portion 14 of the shaft 10. A protruding portion 83 extending spirally and protruding radially outward of the main body portion 81 is provided on a surface of the main body portion 81 of the distal tip 80. The protruding portion 83 is provided in a range from the distal end side of the enlarged diameter portion 82 to a rear end of the enlarged diameter portion 82. According to the distal tip 80, since the protruding portion 83 is provided from the distal end side of the enlarged diameter portion 82, it is possible to further improve the penetrability into a hard lesion site.

The technique disclosed herein may be modified into various modes without departing from the scope of the above-described embodiments and the modification. For example, the following modifications can be applied.

In the above-described embodiment, the shaft 10 includes the coil body 11 as an example of a reinforcing body of the shaft 10. For example, a braid may be employed as the reinforcing body. If a braid is used as the reinforcing body, the braid and the distal tips 30, 40, and 50 may be joined by welding.

For example, in the above-described fourth embodiment, a slit is provided in the distal tip 30 according to the first embodiment (not covered by the subject-matter of the claims). In the above-described fifth embodiment, a protruding portion is provided for the distal tip 30 according to the first embodiment. For example, the distal tip 50 may also be formed with a slit or a protruding portion.

### REFERENCE SIGN LIST

1, 1A, 1B Catheter
10 Shaft
11 Coil body (reinforcing body)
12 Outer layer
13 Inner layer
14 Distal end portion
20 Connector
30, 40, 50, 60, 70, 80 Distal tip
31, 41, 51, 61, 71, 81 Main body portion
32, 42, 52, 62 Coating layer
33, 43, 53, 63, 72, 82 Enlarged diameter portion
44, 54 Outer peripheral surface
64 Slit
73, 83 Protruding portion
R Region
DT1, DT2, DT3, DT4 Outer diameter of enlarged diameter portion
DS Outer diameter of distal end portion

## Claims

1. A catheter (1B), comprising:
a hollow shaft (10); and
a distal tip (50) formed of metal and connected to a distal end portion (14) of the hollow shaft (10), wherein
the distal tip (50) includes, between a distal end of the distal tip (50) and the distal end portion (14) of the hollow shaft (10), an enlarged diameter portion (53) where an outer diameter (DT3) in a direction perpendicular to an axial direction of the hollow shaft (10) is largest;
**characterized in that**
the outer diameter (DT3) of the enlarged diameter portion (53) is larger than an outer diameter (DS) of the distal end portion (14) of the hollow shaft (10); and
the distal tip (50) is formed so that an outer shape (54) from the enlarged diameter portion (53) to the distal end portion (14) of the hollow shaft (10) is outwardly concave.

2. The catheter (1B) according to claim 1, wherein a slit is formed in an outer peripheral surface (54) of the distal tip (50) on the distal end side of the enlarged diameter portion (53).

3. The catheter (1B) according to claim 1 or 2, wherein the outer peripheral surface (54) of the distal tip (50) is formed with a protruding portion that extends spirally and protrudes radially outward.

## Patentansprüche

1. Katheter (1B) umfassend:
einen Hohlschaft (10); und
eine distale Spitze (50), die aus Metall gebildet ist und mit einem distalen Endabschnitt (14) des Hohlschafts (10) verbunden ist, wobei
die distale Spitze (50) zwischen einem distalen Ende der distalen Spitze (50) und dem distalen Endabschnitt (14) des Hohlschafts (10) einen Abschnitt (53) mit vergrößertem Durchmesser aufweist, wo ein Außendurchmesser (DT3) in einer Richtung senkrecht zu einer axialen Richtung des Hohlschafts (10) am größten ist;
**dadurch gekennzeichnet, dass**
der Außendurchmesser (DT3) des Abschnitts (53) mit vergrößertem Durchmesser größer ist als ein Außendurchmesser (DS) des distalen Endabschnitts (14) des Hohlschafts (10); und
die distale Spitze (50) so gebildet ist, dass eine Außenform (54) vom Abschnitt (53) mit vergrößertem Durchmesser zum distalen Endabschnitt (14) des Hohlschafts (10) nach außen konkav ist.

2. Katheter (1B) gemäß Anspruch 1, wobei ein Schlitz in einer Außenumfangsfläche (54) der distalen Spitze (50) auf der distalen Endseite des Abschnitts (53) mit vergrößertem Durchmesser gebildet ist.

3. Katheter (1B) gemäß Anspruch 1 oder 2, wobei die Außenumfangsfläche (54) der distalen Spitze (50) mit einem vorstehenden Abschnitt gebildet ist, der sich spiralförmig erstreckt und radial nach außen vorsteht.

## Revendications

1. Cathéter (1B), comprenant :
un arbre creux (10) ; et
une pointe distale (50) formée de métal et reliée à une partie d'extrémité distale (14) de l'arbre creux (10), dans lequel
la pointe distale (50) inclut, entre une extrémité distale de la pointe distale (50) et la partie d'extrémité distale (14) de l'arbre creux (10), une partie de diamètre agrandi (53) où un diamètre extérieur (DT3) dans une direction perpendiculaire à une direction axiale de l'arbre creux (10) est le plus grand ;
**caractérisé en ce que**
le diamètre extérieur (DT3) de la partie de diamètre agrandi (53) est plus grand qu'un diamètre extérieur (DS) de la partie d'extrémité distale (14) de l'arbre creux (10) ; et
la pointe distale (50) est formée de telle sorte qu'une forme extérieure (54) de la partie de diamètre agrandi (53) à la partie d'extrémité distale (14) de l'arbre creux (10) soit concave vers l'extérieur.

2. Cathéter (1B) selon la revendication 1, dans lequel une fente est formée dans une surface périphérique externe (54) de la pointe distale (50) sur le côté d'extrémité distale de la partie de diamètre agrandi (53).

3. Cathéter (1B) selon la revendication 1 ou 2, dans lequel la surface périphérique externe (54) de la pointe distale (50) est formée avec une partie saillante qui s'étend en spirale et fait saillie radialement vers l'extérieur.
